# EUROPEAN PATENT APPLICATION

(11) **EP 4 524 234 A1**
(43) Date of publication of application: **19.03.2025**
(21) Application number: 24167534.7
(22) Date of filing: 28.03.2024
(51) Int. Cl.: C12N 1/20, C12N 9/00, C12N 9/02, C12N 9/04, C12N 9/06, C12N 9/08, C12N 9/10, C12N 9/12, C12N 9/14, C12N 9/88, C12P 7/22, C12P 7/24

(54) **GENETICALLY ENGINEERED BACTERIUM USING GLUCOSE AS SUBSTRATE FOR DE NOVO SYNTHESIS OF VANILLIN AND APPLICATION THEREOF**

(30) Priority: 14.09.2023 CN 202311181895
(71) Applicant: Beijing University Of Chemical Technology, Beijing 100029 (CN)
(72) Inventor: TAN, Tianwei, Beijing 100029 (CN); YU, Yingyue, Beijing 100029 (CN); ZHU, Haowen, Beijing 100029 (CN); LONG, Jianyu, Beijing 100029 (CN)
(74) Representative: Valet Patent Services Limited

(57) **Abstract**

The present invention discloses a genetically engineered bacterium using glucose as a substrate for de novo synthesis of vanillin and an application thereof, which belongs to the technical field of gene recombination and metabolic engineering. The genetically engineered bacterium using the glucose as the substrate for de novo synthesis of vanillin disclosed by the present invention is recombinant *Corynebacterium glutamicum* modified by chassis microorganisms and including a vanillin synthesis module and a methyl cyclic regeneration module. The genetically engineered bacteria constructed by the present invention are safe and non-toxic, can use the glucose for de novo synthesis of natural vanillin, and is low in production cost, high in yield, and promising in application prospect.

## Description

### TECHNICAL FIELD

The present invention relates to the technical field of gene recombination and metabolic engineering, and more particularly to a genetically engineered bacterium using glucose as a substrate for de novo synthesis of vanillin and an application thereof.

### BACKGROUND

Vanillin is an aromatic compound with hydroxyl, aldehyde and oxymethyl substituent groups; and the CAS number is 121-33-5, the molecular formula is C₈H₈O₃, and the molecular weight is 152.15. The vanillin is usually a white or light yellow crystal with a melting point of 81-83°C and a boiling point of 284-285°C, and is difficult to dissolve in water and soluble in organic solvents such as methanol. As an important aromatic compound, the vanillin is wide in use. The vanillin can not only be used as a food additive for a variety of foods in life, but also can be used in cosmetics such as perfume and daily necessities such as soap and toothpaste to stabilize and enhance fragrance. Furthermore, the vanillin is also used for producing products such as plastics, rubber sterilizing agents, electroplating agents, conductive agents and the like in industry. In recent years, the vanillin has been widely used in the medical field to produce various pharmaceutical intermediates, and vanillin may become the most valuable aromatic compound in the medical field.

As a big producer and user of vanillin, the annual demand of the vanillin in China reaches 2350 tons, and the demand still increases year by year. The vanillin produced in China is not only for domestic use, but also is exported to Europe, America, Southeast Asia and other countries, enjoying a good reputation in the world. Based on the above situation, producing the vanillin by biological fermentation is a promising production method, which can more efficiently obtain the compound from cheap sugar. It is already reported in multiple literatures that the vanillin is synthesized by microbial fermentation, but the microbial fermentation has the defect of low yield. The biological de novo synthesis of vanillin was first reported in the de novo synthesis of vanillin in *Schizosaccharomyces pombe* and *Saccharomyces cerevisiae* with protocatechuic acid as an intermediate, but the yield of the vanillin was only 65 mg/L and 45 mg/L (Hansen EH, et al. De novo biosynthesis of vanillin in fission yeast (Schizosaccharomyces pombe) and baker's yeast (Saccharomyces cerevisiae).[J].Applied & Environmental Microbiology, 2009, 75(9):2765-74.). It was reported by Ni, et al. that 19.3 mg/L vanillin was synthesized de novo in *Escherichia coli* with ferulic acid as an intermediate and glucose as a substrate, but the yield of vanillin was still very low. (Ni J, et al. Mimicking a natural pathway for de novo biosynthesis: natural vanillin production from accessible carbon sources[J]. Rep, 2015, 5(1):13670.). Chen Z, et al. reported that 240.69 mg/L of vanillyl alcohol was synthesized de novo in *Escherichia coli* (Chen Z, et al. Establishing an Artificial Pathway for De Novo Biosynthesis of Vanillyl Alcohol in Escherichia coli[J]. Acs Synthetic Biology, 2017: acssynbio.7b00129.). Then Yang et al. reported that 559.4 mg/L of vanillyl alcohol was synthesized de novo in a shake flask with glucose as a substrate in a way of *Escherichia coli* mix (Yang M, Meng H, Li X, et al. Coculture engineering for efficient production of vanillyl alcohol in Escherichia coli[J].aBIOTECH, 2022, 3(4):9.), but the vanillyl alcohol is an aldehyde reduction product of vanillin, and has quite different product characteristics from vanillin. In recent years, Kim et al. reported that 310 mg/L vanillin was synthesized de novo in *Corynebacterium glutamicum* from glucose via an intermediate hydroxybenzoic acid, the de novo synthesis yield of vanillin was greatly increased (Kim H S, et al. Engineered Corynebacterium glutamicum as the Platform for the Production of Aromatic Aldehydes[J]. Frontiers in Bioengineering and Biotechnology, 2022, 10: 880277.), but there is still a certain gap from industrial scale-up production.

Therefore, providing a genetically engineered bacterium using glucose as a substrate for de novo synthesis of vanillin and an application thereof is an urgent problem to be solved by those skilled in the art.

### SUMMARY

In view of this, the present invention provides a genetically engineered bacterium using glucose as a substrate for de novo synthesis of vanillin and an application thereof, which researches and develops an efficient pathway of biological de novo synthesis of vanillin for problems of the existing biological de novo synthesis of vanillin, and constructs a non-toxic food safe engineered strain capable of using cheap substrates such as glucose to produce the natural vanillin.

A purpose of the present invention is to provide a genetically engineered bacterium for synthesizing vanillin. The strain is safe and non-toxic and can use a microbial fermentation method to produce the vanillin, which has low production cost.

Specifically, a genetically engineered bacterium using glucose as the substrate for de novo synthesis of vanillin is provided; and the genetically engineered bacterium is a recombinant *Corynebacterium glutamicum* modified by genome and including a vanillin synthesis module and a methyl cyclic regeneration module.

The modification of chassis microorganism is to take *Corynebacterium glutamicum* as an original strain to knock out *pcaHG* (protocatechuate 3,4-dioxygenase subunit, protocatechuate 3,4-dioxygenase subunit beta (pcaH), protocatechuate 3,4-dioxygenase subunit alpha (pcaG)), *van* (vanillate demethylase), *vdh* (vanillin dehydrogenase) and *fud* (alcohol dehydrogenase (NADP+), and Zn-dependent alcohol dehydrogenases) genes.

The vanillin synthesis module expresses a transketolase gene, a 3-deoxy-7-phosphoheptulonate synthase gene, an O-methyltransferase gene, a Carboxylic acid reductase gene and a 4'-phosphopantetheinyl transferase gene.

The transketolase can efficiently enhance the synthesis of E4P in an HMP pathway, and increase the yield of DAHP. The 3-deoxy-7-phosphoheptulonate synthase helps PEP and E4P to synthesize DAHP. The O-methyltransferase catalyzes protocatechuic acid to generate vanillic acid. The Carboxylic acid reductase catalyzes the vanillic acid to be reduced into vanillin. Because the carboxylic acid reductase expressed by *Corynebacterium glutamicum* is an apoenzyme, the carboxylic acid reductase becomes a whole enzyme under the translational phosphorylation modification of 4'-phosphopantetheinyl transferase.

The methyl cyclic regeneration module expresses a 5-methyltetrahydropteroyltriglutamate-homocysteine methyltransferase gene, an S-adenosylmethionine synthase gene, a homoserine O-acetyltransferase gene and an adenosylhomocysteinase gene.

The homoserine O-acetyltransferase transfers acyl groups of coenzyme A (CoA) or succinyl-CoA to hydroxyl oxygen of homoserine, so that the homoserine is acetylated to form O-acetyl-1-homoserine, preparing for subsequent vulcanization. The strain transforms O-acetyl-l-homoserine to L-homocysteine. The 5-methyltetrahydropteroyltriglutamate-homocysteine methyltransferase helps catalyze the transfer of methyl from 5- methyltetrahydrofolate to L-homocysteine, leading to the formation of methionine, and catalyze the reaction between 5- methyl tetrahydropteroyl tri -L- glutamic acid and L-homocysteine to generate L-methionine and tetrahydropteroyl tri-L-glutamic acid. The S-adenosylmethionine synthase ATP catalyzes the reaction between ATP and water and L-methionine to generate diphosphate, phosphate and S-adenosyl-L-methionine. The adenosylhomocysteinase catalyzes the reaction between water and S-adenosyl-L-homocystein to generate adenosine and L-homocysteine.

Further, the transketolase gene includes an endogenous *Corynebacterium glutamicum* transketolase gene *tktA* and an optional exogenous transketolase gene.

The 3-deoxy-7-phosphoheptulonate synthase gene includes an endogenous *Corynebacterium glutamicum*3-deoxy-7-phosphoheptulonate synthase gene *aroG* and an optional exogenous 3-deoxy-7-phosphoheptulonate synthase gene.

The O-methyltransferase gene includes an O-methyltransferase gene *comt* from *Arabidopsis thaliana* (*Mouseear cress)*, an O-methyltransferase gene *comt* from *Coffea canephora (Robusta coffee*), an O-methyltransferase gene *comt* from *Homo sapiens (Human*), an O-methyltransferase gene *comt* from *Rattus norvegicus (rat)*, an O-methyltransferase gene *comt* from *Mus musculus (mouse)*, and an optional O-methyltransferase gene *comt.*

The carboxylic acid reductase gene includes a carboxylic acid reductase gene *car* from *Nocardia iowensis* and a carboxylic acid reductase gene *car* from *Mycobacterium marinum.*

The 4'-phosphopantetheinyl transferase gene includes a 4'-phosphopantetheinyl transferase gene *sfp* from *Nocardia iowensis*, a 4'-phosphopantetheinyl transferase gene *sfp* from *Mycobacterium marinum,* and a 4'-phosphopantetheinyl transferase gene *sfp* from *Bacillus subtilis.*

The 5-methyltetrahydropteroyltriglutamate-homocysteine methyltransferase gene includes an endogenous *Corynebacterium glutamicum* 5-methyltetrahydropteroyltriglutamate-homocysteine methyltransferase gene *metE*, and an optional exogenous 5-methyltetrahydropteroyltriglutamate-homocysteine methyltransferase gene.

The S-adenosylmethionine synthase gene includes an endogenous *Corynebacterium glutamicum* S-adenosylmethionine synthase gene *metK* and an optional exogenous S-adenosylmethionine synthase gene.

The homoserine O-acetyltransferase gene includes an endogenous *Corynebacterium glutamicum* homoserine O-acetyltransferase gene *metX* and an optional exogenous homoserine O-acetyltransferase gene.

The adenosylhomocysteinase gene includes an endogenous *Corynebacterium glutamicum* adenosylhomocysteinase gene *ahcY* and an optional exogenous adenosylhomocysteinase gene.

The nucleotide sequence of the endogenous *Corynebacterium glutamicum* transketolase gene *tktA* is as shown by SEQ ID NO. 83.

The nucleotide sequence of the endogenous *Corynebacterium glutamicum* 3-deoxy-7-phosphoheptulonate synthase gene *aroG* is as shown by SEQ ID NO. 84.

The nucleotide sequence of the O-methyltransferase gene *comt* from *Arabidopsis thaliana* is as shown by SEQ ID NO. 85; the nucleotide sequence of the O-methyltransferase gene *comt* from *Coffea canephora* is as shown by SEQ ID NO. 122; the nucleotide sequence of the O-methyltransferase gene *comt* from *Homo sapiens* is as shown by SEQ ID NO. 105; the nucleotide sequence of the O-methyltransferase gene *comt* from *Rattus norvegicus* is as shown by SEQ ID NO. 46; and the nucleotide sequence of the O-methyltransferase gene *comt* from *Mus musculus* is as shown by SEQ ID NO. 137.

The nucleotide sequence of the carboxylic acid reductase gene *car* from *Nocardia iowensis* is as shown by SEQ ID NO. 47; and the nucleotide sequence of the carboxylic acid reductase gene *car* from *Mycobacterium marinum* is as shown by SEQ ID NO. 86.

The nucleotide sequence of the 4'-phosphopantetheinyl transferase gene *sfp* from *Nocardia iowensis* is as shown by SEQ ID NO. 87; and the nucleotide sequence of the 4'-phosphopantetheinyl transferase gene *sfp* from *Mycobacterium marinum* is as shown by SEQ ID NO. 48; and the nucleotide sequence of the 4'-phosphopantetheinyl transferase gene *sfp* from *Bacillus subtilis* is as shown by SEQ ID NO. 106.

The nucleotide sequence of the endogenous *Corynebacterium glutamicum* 5-methyltetrahydropteroyltriglutamate-homocysteine methyltransferase gene *metE* is as shown by SEQ ID NO. 49.

The nucleotide sequence of the endogenous *Corynebacterium glutamicum* S-adenosylmethionine synthase gene *metK* is as shown by SEQ ID NO. 50.

The nucleotide sequence of the endogenous *Corynebacterium glutamicum* homoserine O-acetyltransferase gene *metX* is as shown by SEQ ID NO. 51.

The nucleotide sequence of the endogenous *Corynebacterium glutamicum* adenosylhomocysteinase gene *ahcY* is as shown by SEQ ID NO. 52.

Further, the exogenous transketolase gene is derived from *Escherichia coli* K12 (*tktA*, Gene ID: 947420), *Saccharomyces cerevisiae* S288C (*tk11*, Gene ID: 856188) or *Bacillus subtilis* 168 (*tktA*, Gene ID: 937377).

The exogenous 3-deoxy-7-phosphoheptulonate synthase gene is derived from *Escherichia coli* K12 (the nucleotide sequence is as shown by SEQ ID NO. 45), *Saccharomyces cerevisiae* S288C (*aro4*, Gene ID: 852551) or *Bacillus subtilis* 168 (*aroX*, Gene ID: 937853).

The exogenous 5-methyltetrahydropteroyltriglutamate-homocysteine methyltransferase gene is derived from *Escherichia coli* K12 (*metE*, Gene ID: 948323), *Saccharomyces cerevisiae* S288C (*met6*, Gene ID: 856825) or *Bacillus subtilis* 168 (*metE*, Gene ID: 936480).

The exogenous S-adenosylmethionine synthase gene is derived from *Escherichia coli* K12 (*metK*, Gene ID: 948323), *Saccharomyces cerevisiae* S288C (*SAM2*, Gene ID: 852113) or *Bacillus subtilis* 168 (*metK*, Gene ID: 937090);

The exogenous homoserine O-acetyltransferase gene is derived from *Saccharomyces cerevisiae* S288C (*met2*, Gene ID: 855444) or *Bacillus subtilis* 168 (*metAA*, Gene ID: 939083).

Preferably, the exogenous transketolase gene is derived from *Escherichia coli* K12.

The exogenous 3-deoxy-7-phosphoheptulonate synthase gene is derived from *Escherichia coli* K12.

The O-methyltransferase gene is derived from *Rattus norvegicus.*

The carboxylic acid reductase gene is derived from *Nocardia iowensis.*

The 4'-phosphopantetheinyl transferase gene is derived from *Mycobacterium marinum.*

The 5-methyltetrahydropteroyltriglutamate-homocysteine methyltransferase gene is derived from endogenous *Corynebacterium glutamicum.*

The S-adenosylmethionine synthase gene is derived from the endogenous *Corynebacterium glutamicum.*

The homoserine O-acetyltransferase gene is derived from the endogenous *Corynebacterium glutamicum.*

The adenosylhomocysteinase gene is derived from the endogenous *Corynebacterium glutamicum.*

Further, a method for constructing a genetically engineered bacterium using glucose as a substrate for de novo synthesis of vanillin includes the following steps:
(1) using *Corynebacterium glutamicum* as an original strain to knock out *pcaHG*, *van*, *vdh* and *fud* genes, and obtaining modified *Corynebacterium glutamicum*;
(2) expressing the vanillin synthesis module and the methyl cyclic regeneration module in the modified *Corynebacterium glutamicum.*

Another purpose of the present invention is to provide an application of the genetically engineered bacterium for synthesizing vanillin in synthesis of vanillin.

Specifically, the present invention provides an application of the genetically engineered bacterium or the method in producing vanillin.

Specifically, the present invention provides an application of the genetically engineered bacterium or the method in increasing a yield of vanillin.

Specifically, a method for producing the vanillin uses the genetically engineered bacterium or the genetically engineered bacterium constructed by the method for fermentation.

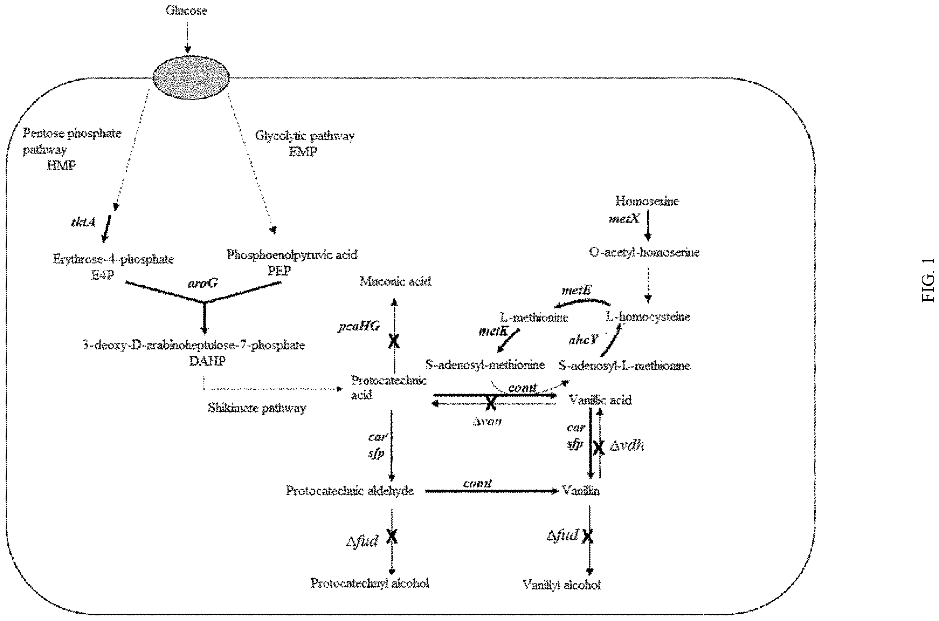

Therefore, the present invention provides a pathway for biosynthesis of vanillin (Fig. 1), which includes:
(1) glucose generates phosphoenolpyruvate (PEP) under the action of a glycolytic pathway of microorganisms, and generates erythrose 4-phosphate (E4P) under the action of a pentose phosphate pathway of microorganisms;
(2) transketolase tktA catalyzes reversible transfer of dicarbonyl groups from 7-heptose phosphate to glyceraldehyde 3-phosphate to generate 5-phosphoxylose and ribose 5-phosphate;
(3) the phosphoenolpyruvate (PEP) and the erythrose 4-phosphate (E4P) are catalyzed by 3-deoxy-D-arabinoheptulose-7-phosphate synthase aroG to generate 3-deoxy-D-arabinoheptulose-7-phosphate (DAHP);
(4) *Corynebacterium glutamicum* generates protocatechuic acid (PCA) in a pathway of own shikimic acid;
(5) the protocatechuic acid (PCA) is catalyzed by O-methyltransferase comt to generate vanillic acid;
(6) the vanillic acid is catalyzed by carboxylic acid reductase car to generate vanillin;
(7) the carboxylic acid reductase car is transformed into a whole enzyme by post-translational modification of catalytic phosphorylation of 4'-phosphopantetheinyl transferase sfp;
   the above is the vanillin synthesis module.
(8) strain endogenously generates homoserine;
(9) homoserine O-acetyltransferase metX transfers an acetyl group from coenzyme A to l-homoserine to form O-acetyl-l-homoserine;
(10) the strain transforms O-acetyl-l-homoserine to L-homocysteine;
(11) the 5-methyltetrahydropteroyltriglutamate-homocysteine methyltransferase metE catalyzes the transfer of a methyl group from 5-methyltetrahydrofolate to homocysteine to form methionine;
(12) the L-methionine is catalyzed by S-adenosylmethionine synthase metK to generate S-adenosyl-L-methionine (SAM);
(13) the S-adenosyl-L-methionine is catalyzed by O-methyltransferase comt to provide a methyl group for protocatechuic acid to generate S-adenosyl-L-homocystein (SAH) and vanillic acid;
(14) the S-adenosyl-L-homocystein is catalyzed by adenosylhomocysteinase ahcY to generate adenosine and L-homocysteine;
   the above is the methyl cyclic regeneration module.

Modification of chassis microorganisms: *Corynebacterium glutamicum* is used as an original strain to knock out *pcaHG*, *van*, *vdh and fud* genes with specific steps as follows:

*pcaHG* is a protocatechuate 3,4-dioxygenase subunit; the *Corynebacterium glutamicum* has a pathway of β -ketoadipic acid, and uses several aromatic compounds such as PCA as an exclusive source of carbon and energy; the degradation of protocatechuic acid is initiated by protocatechuate 3,4-dioxygenase (*PcaGH*), which consists of two subunits coded by *pcaG* and *pcaH* located in a *pcaHGBC* operon; and knocking out *pcaHG* can prevent the consumption of the protocatechuic acid β -ketoadipic acid pathway.

*Van* is vanillic demethylase and contains vanillic demethylase subunit ABK, which can catalyze the transformation of vanillic acid to protocatechuic acid; and knocking out *van* can prevent demethylation from vanillic acid to protocatechuic acid, thereby promoting the utilization of the protocatechuic acid.

*vdh* catalyzes the oxidation of vanillin to generate vanillic acid, which has an obvious oxidation effect on 3,4-dihydroxybenzaldehyde (protocatechuic aldehyde) and 4-hydroxybenzaldehyde, and also has activities of NAD+ and NADP+. Due to the toxicity of vanillin for cells, *vdh* catalyzes the transformation of vanillin to vanillic acid; and knocking out *vdh* can reduce the oxidation of vanillin and increase the accumulation of vanillin.

*Fud* is an alcoholdehydrogenase, which uses NADPH (not NADH) as a cofactor to transform vanillin into vanillyl alcohol; and knocking out Fud can significantly reduce the process of reducing vanillin to vanillyl alcohol.

It may be seen from the above technical solution that compared with the prior art, the present invention discloses the genetically engineered bacterium using the glucose as the substrate for de novo synthesis of vanillin and the application thereof, which modifies the *Corynebacterium glutamicum* in three aspects, including construction and enhancement of a vanillin synthesis pathway (involving *tktA*, *aroG*, *comt* and *car*), enhancement of a methyl regeneration system (involving *metE*, *metK*, *metX*, and *ahcY*), knocking out of a vanillin catabolism pathway and a byproduct metabolism pathway (involving *pcaHG*, *van*, *vdh* and *fud*); and finally a recombinant *Corynebacterium glutamicum* for efficiently synthesizing the vanillin is constructed.

The host *Corynebacterium glutamicum* used in the present invention is a food-grade microorganism, which has no pathogenicity to humans and animals, has better growth advantages under the same culture conditions, and can accumulate higher concentration of vanillin than other strains. Repeated experiments show that the highest concentration of vanillin produced by the shaking flask fermentation of recombinant *Corynebacterium glutamicum* can reach 765.85 mg/L, which is the highest yield of vanillin of de novo biosynthesis in the reported related research at present, and has great industrial application prospects in the biological production of vanillin with high yield.

The genetically engineered bacterium constructed by the present invention is safe and non-toxic, can use the microbiological fermentation method to produce the vanillin with the glucose as the substrate, and is simple in culture medium components, stable in batches, and low in production cost.

### DESCRIPTION OF DRAWINGS

To more clearly describe the technical solutions in the embodiments of the present invention or in the prior art, the drawings required to be used in the description of the embodiments or the prior art will be simply presented below. Apparently, the drawings in the following description are merely embodiments of the present invention, and for those ordinary skilled in the art, other drawings can also be obtained according to the provided drawings without contributing creative labor.
Fig. 1 is a pathway of biological synthesis of vanillin according to the present invention.
Fig. 2 shows a yield and biomass OD₆₀₀ of vanillin synthesized by genetically engineered bacteria constructed in embodiment 1 of the present invention by shaking flask fermentation with glucose as substrate at different times.
Fig. 3 shows a yield and biomass OD₆₀₀ of vanillin synthesized by genetically engineered bacteria constructed in embodiment 2 of the present invention by shaking flask fermentation with glucose as substrate at different times.
Fig. 4 shows a yield and biomass OD₆₀₀ of vanillin synthesized by genetically engineered bacteria constructed in embodiment 3 of the present invention by shaking flask fermentation with glucose as substrate at different times.
Fig. 5 shows a yield and biomass OD₆₀₀ of vanillin synthesized by genetically engineered bacteria constructed in embodiment 4 of the present invention by shaking flask fermentation with glucose as substrate at different times.
Fig. 6 shows a yield and biomass OD₆₀₀ of vanillin synthesized by genetically engineered bacteria constructed in embodiment 5 of the present invention by shaking flask fermentation with glucose as substrate at different times.

### DETAILED DESCRIPTION

Technical solutions in the embodiments of the present invention are described clearly and fully below in combination with the drawings in the embodiments of the present invention. Apparently, the described embodiments are merely part of the embodiments of the present invention, not all of the embodiments. Based on the embodiments in the present invention, all other embodiments obtained by those ordinary skilled in the art without contributing creative labor will belong to the protection scope of the present invention.

LBHIS liquid culture medium: peptone 5.0 g/L, yeast powder 2.5 g/L, NaCL 5.0 g/L, brain heart infusion (BHI) 18.5 g/L, and sorbitol 91.0 g/L. 1.8%-2% of agar is added to the corresponding LBHIS solid culture medium is added with The LBHIS culture medium is mainly used for culturing *Corynebacterium glutamicum* in a test tube and a solid plate.

EPO liquid culture medium: peptone 10.0 g/L, yeast powder 5.0 g/L, NaCL 10.0 g/L, glycine 30.0 g/L, and Tween80 10.0 g/L. The EPO culture medium is mainly used for preparing *Corynebacterium glutamicum* competent cells.

30% sucrose culture medium: peptone 10.0 g/L, yeast powder 5.0 g/L, NaCL 10.0 g/L, and sucrose 300.0 g/L.

20% sucrose solid culture medium: peptone 10.0 g/L, yeast powder 5.0 g/L, NaCL 10.0 g/L, sucrose 200.0 g/L, and agar 15.0 g/L. LB-suc culture medium (30% sucrose culture medium, and 20% sucrose solid culture medium) is mainly used for homologous recombination and screening in a *Corynebacterium glutamicum* gene knockout experiment.

LBG culture medium: peptone 10.0 g/L, yeast powder 5.0 g/L, NaCL 10.0 g/L and glucose 20.0 g/L. The LBG culture medium is mainly used as a seed culture medium in fermentation of *Corynebacterium glutamicum.*

Fermentation culture medium: glucose 65.0 g/L, urea 5.0 g/L, maize extract powder 8.0 g/L, biotin 4×10⁻⁴ g/L, VB₁ biotin 4×10⁻⁴ g/L, K₂HPO₄ 1.0 g/L, KH₂PO₄ 1.0 g/L, CaCl₂•2H₂O 29.4 mg/L, MgSO₄•7H₂O 1.2325 g/L, and trace element solution 0.2%. (A preparation method of the trace element solution: measuring 1 g of FeSO₄·7H₂O, 1g of MnSO₄·H₂O, 0.1g of ZnSO₄·7H₂O, 0.2g of CuSO₄, and 0.002g of NiCl₂·6H₂O, adding water to fix the volume at 100 mL, adding 100 µl of concentrated hydrochloric acid to adjust pH, then filtering bacteria, and adding 0.2% in a fermentation culture medium system). The fermentation culture medium is mainly used for the shake-flask fermentation of *Corynebacterium glutamicum.*

### Embodiment 1

A method for constructing a genetically engineered bacterium using glucose as a substrate for de novo synthesis of vanillin includes the following steps:

With genome DNA of *Corynebacterium glutamicum* ATCC13032 as a template, pcaHG-L (as shown by SEQ ID NO.1) is amplified with primers pcaHG-L-up/pcaHG-L-down, and pcaHG-R (as shown by SEQ ID NO. 2) is amplified with primers pcaHG-R-up/pcaHG-R-down to obtain an upstream and a downstream homologous arms with all pcaHG deleted. The primer sequences are as follows:
pcaHG-L-up: 5'-ATGATTACGCCATCGCATTGCCGAAAAGC-3'; SEQ ID NO. 3;
pcaHG-L-down: 5'-GGTCAATGCGAGACCTTTCTGCGTC-3'; SEQ ID NO. 4;
pcaHG-R-up: 5'-AAAGGTCTCGCATTGACCCGATCTTTATACTCCGAC-3'; SEQ ID NO. 5;
pcaHG-R-down: 5'-CTCAACGTTGACGGTGATGCCA-3'; SEQ ID NO. 6.

Gene amplification system: 25 µL of Primestar (Takara), 2µL of each of upstream and downstream primers, 1 µL of template, and 20 µL of ddH₂O.

Gene amplification procedure: predegenation at 98°C for 3 min; degeneration at 98°C for 10 se; annealing at 55°C for 30 s; extension 10 s/kb for 35 cycles at 72°C; and extension at 72°C for 10 min.

PCR products are subjected to agarose gel electrophoresis and product recovery to obtain gene fragments pcaHG-L and pcaHG-R.

Plasmid vector pk18mobsacb is linearized with the primers pk-pcaHG-F and pk-pcaHG-R to obtain a pk-pcaHG vector, and the primer sequences are as follows:
pk-pcaHG-F: 5'-ATCACCGTCAACGTTGAGCTCGGTAGATCCTCTAGAGT-3'; SEQ ID NO. 7;
pk-pcaHG-R:5'-AATGCGATGGCGTAATCATGTCATAGCTGTTTCCTG-3'; SEQ ID NO. 8.

3 µL of pcaHG-L vector, 3 µL of pcaHG-R vector, 4 µL of pk-pcaHG vector and 10.0 µl of Gibson ligase are added into a PCR tube, wherein the ligation temperature is 50°C, the ligation time is 15 min, and the total system is 20 µL.

20 µl of total ligation system of Gibson ligation is transformed to *E.coli* Trans10 commercial competence (TransGen Biotech) for culture and preservation. The transformation operation is strictly carried out according to instructions; after cultured at 37°C for 1 h, the whole system is coated on an LB plate (containing 50µg/mL kanamycin), then the whole system is cultured at 37°C for 12 h, and about 10 to 20 single colonies are selected for colony PCR amplification and DNA sequencing verification. Primers for colony PCR amplification and DNA sequencing are as follows:
Pklj-F: 5'-GCGGATAACAATTTCACACAGGA-3'; SEQ ID NO. 9;
Pklj-R: 5'-cgggcctcttcgctattac-3'; SEQ ID NO. 10.

A correct single colony is selected, and named *Escherichia coli* WJ001; and after propagation and plasmid extraction, WJ001 plasmid is obtained. The *Escherichia coli* WJ001 plasmid is electrotransformed into competence of *Corynebacterium glutamicum* ATCC13032.

Competence cells of *Corynebacterium glutamicum* ATCC13032 are prepared as follows: glycerol preserved strains of *Corynebacterium glutamicum* ATCC13032 are selected, streaked and inoculated onto an LBHIS plate and cultured for about 24-30 h in an incubator at 30°C; colonies are selected from the plate and inoculated into a test tube with LBHIS liquid culture medium to be cultured for 12 h, and inoculated into 20 ml EPO culture medium at a certain inoculation amount, so that an initial OD₆₀₀ is about 0.3, the colonies are subjected to shake-flask culture for about 3-5 h at 30°C, and when OD₆₀₀ reaches about 0.9, bacterial solution is collected and placed into a centrifugal tube, and subjected to ice-bath for 15 min to cool the bacteria cells to 0°C; after frozen centrifuging for 10 min at 4500 rpm and 4°C, the bacterial cells are collected (split charged in 1.5 mL centrifugal tubes), supernatant is removed, and the bacterial cells are re-suspended with 100µL of pre-cooled 10% sterile glycerol (three tubes of bacterial cells are mixed into one); the previous step is repeated three times; after washing, the bacterial cells are re-suspended by 100 µL of sterile glycerol, and then split charged and directly used for electrotransformation.

The electrotransformation method is as follows: 2-4 µL of plasmid is added into each tube of competent cells, uniformly mixed and ice-bathed for 5-10 min; and the mixed solution is transferred into a pre-cooled 0.2 cm electric shock cup for electric shock for 5 ms with 1.8 kv, 50µF and 100Ω. After the electric shock, the mixed solution is immediately added to 800µL LBHIS liquid culture medium and slightly mixed, then the mixed solution is sucked into a 1.5 mL centrifugal tube for water bath or metal bath for 6 min at 46°C, and then cultured at 30°C for 2-3 h; and the mixed solution is centrifuged for 1.5 min at 8000 rpm, supernatant is removed, and about 100-200 µl of bacterial solution is preserved and uniformly mixed and coated on a LBHIS solid culture medium plate containing 50µg/mL kanamycin and cultured at 30°C for 24-36 h.

The single colony growing on the plate is selected to verify a *sacB* gene, and only the colony that can grow on a Kana-resistant plate and also can amplify *sacB* gene (a band size is 999 bp) is the strain with the first homologous recombination. The verification primers are as follows:
sac-F: 5'-CCCATATTACACGCCATGATATGCT-3'; SEQ ID NO. 11;
sac-R: 5'-GCATGTAAATATCGTTAGACGTAATGCCG-3'; SEQ ID NO. 12.

The strain that is verified to be correct is selected and inoculated into 30% sucrose culture medium to be cultured for 24 h, and after the bacterial solution is turbid, the bacterial solution is streaked on the 20% sucrose solid culture medium; the single colony on the plate is selected for colony PCR of *sacB* genes; and the single colonies that do not amplify the sacB genes are subjected to secondary colony PCR with verification primers, and the pcaHG verification (the band size is 3376 bp) primers are as follows:
pcaHG-verification-F: 5'-CGCGACTTGCCATCACATC-3'; SEQ ID NO. 13;
pcaHG-verification-R: 5'-GTCAAGCAGGCTAAACCGGAG-3'; SEQ ID NO. 14.

According to the knock-out principle of *Corynebacterium glutamicum*, after the second homologous recombination, the whole knock-out plasmid may fall off from the genome, so as to achieve the purpose of knock-out, so that the colony with the second recombination may no longer contain *sacB* genes; the bacteria that is verified by the verification primers to succeed in knock-out are inoculated in the LBHIS liquid medium for culture, and then streaked on the LBHIS solid medium plate for purification and re-verification; and after confirmation, the bacteria can be preserved for use, and cgATCC13032Δ*pcaHG* is named *Corynebacterium glutamicum* CG001.

(2) With genome DNA of *Corynebacterium glutamicum* ATCC13032 as a template, van-L (as shown by SEQ ID NO.15) is amplified with primers van-L-up/van-L-down, and van-R (as shown by SEQ ID NO. 16) is amplified with primers van-R-up/van-R-down to obtain an upstream and a downstream homologous arms with all van deleted. The primer sequences are as follows:
van-L-up:5'-AACAGCTATGACATGATTACGATAAGCTGCTCATGCTTGGC-3'; SEQ ID NO. 17;
van-L-down:5'-CTGATATCTCGACATTGCTGAGTTAATGTCAGCTGGGGTC TC-3'; SEQ ID NO. 18;
van-R-up: 5'-CAGCAATGTCGAGATATCAGTGG-3'; SEQ ID NO. 19;
van-R-down:5'-AGAGGATCTACCGAGCTCTCATCAGTGTGTTTCGGGAGC-3'; SEQ ID NO. 20.

The plasmid vector PK-JL is linearized with the primers pk-van-F and pk-van-R to obtain a pk-van vector, and the primer sequences are as follows:
pk-van-F: 5'-GAGCTCGGTAGATCCTCTAGAGT-3'; SEQ ID NO. 21;
pk-van-R: 5'-CGTAATCATGTCATAGCTGTTTCCTG-3'; SEQ ID NO. 22.

The gene amplification system and the gene amplification procedure are the same as above, and van-L, van-R and pk-van vectors are obtained; the verification method of ligation and transformation is the same as above, and *Escherichia coli* WJ002 is obtained; and after propagation and plasmid extraction, WJ002 plasmid is obtained.

The *Escherichia coli* WJ002 plasmid is electrotransformed into competence of *Corynebacterium glutamicum* CG001. The competence preparation, electrotransformation, sacB verification, and culture with sucrose culture medium are the same as above, and the partial deletion van verification (the size band is 4459 bp) primers are as follows:
van-verification-F: 5'-TTGATGGTGTCCGCAAAATCG-3; SEQ ID NO. 23;
van-verification-R: 5'-ACCGACACGACCCATACCAA-3; SEQ ID NO. 24.

The bacteria that are verified with the verification primers to succeed in knock-out are inoculated in LBHIS liquid medium for culture, and then streaked on LBHIS solid medium plate for purification and re-verification; and after confirmation, the bacteria can be preserved for use, and cgATCC13032Δ*pcaHG*Δ*van* is named *Corynebacterium glutamicum* CG002.

(3) With genome DNA of *Corynebacterium glutamicum* ATCC13032 as a template, vdh-L (as shown by SEQ ID NO.25) is amplified with primers vdh-L-up/vdh-L-down, and vdh-R (as shown by SEQ ID NO. 26) is amplified with primers vdh-R-up/vdh-R-down to obtain an upstream and a downstream homologous arms with all *vdh* deleted. The primer sequences are as follows:
vdh-L-up:5'-AACAGCTATGACATGATTACGCGACGGTATTCCTGCAGATG A-3'; SEQ ID NO. 27;
vdh-L-down:5'-CTTTAGGAGACCTTCGCTAATCCGCTGAACAGG-3'; SEQ ID NO. 28;
vdh-R-up: 5'-GATTAGCGAAGGTCTCCTAAAGTTGATTGTGGATAC-3'; SEQ ID NO. 29;
vdh-R-down: 5'-AGCTCAGTATGGGAAGCATTGCTTGC-3'; SEQ ID NO. 30.

The plasmid vector PK-JL is linearized with primers pk-vdh-F and pk-vdh-R to obtain a pk-vdh vector, and the primer sequences are as follows:
pk-vdh-F:5'-AATGCTTCCCATACTGAGCTCGGTAGATCCTCTAGAGT-3'; SEQ ID NO. 31;
pk-vdh -R: 5'-CGTAATCATGTCATAGCTGTTTCCTG-3'; SEQ ID NO. 32.

The gene amplification system and the gene amplification procedure are the same as above, and vdh-L, vdh-R and pk-vdh vectors are obtained; the verification method of ligation and transformation is the same as above, and *Escherichia coli* WJ003 is obtained; and after propagation and plasmid extraction, WJ003 plasmid is obtained.

The *Escherichia coli* WJ003 plasmid is electrotransformed into competence of *Corynebacterium glutamicum* CG002. The competence preparation, electrotransformation, sacB verification, and culture with sucrose culture medium are the same as above, and the vdh verification (the size band is 3653 bp) primers are as follows:
vdh-verification-F: 5'-GTGAATCAATCATTGCTGATTACCTTGTC-3';SEQ ID NO. 33;
vdh-verification-R: 5'-CTTATGCCTTTGCGTAATGTTGATAGAAC-3; SEQ ID NO. 34.

The bacteria that are verified with the verification primers to succeed in knock-out are inoculated in LBHIS liquid medium for culture, and then streaked on LBHIS solid medium plate for purification and re-verification; and after confirmation, the bacteria can be preserved for use, and cgATCC13032Δ*pcaHG*Δ*van*Δ*vdh* is named *Corynebacterium glutamicum* CG003.

(4) Wwith genome DNA of *Corynebacterium glutamicum* ATCC13032 as a template, fud-L (as shown by SEQ ID NO. 35) is amplified with primers fud-L-up/fud-L-down, and fud-R (as shown by SEQ ID NO. 36) is amplified with primers fud-R-up/fud-R-down to obtain an upstream and a downstream homologous arms of fud. The primer sequences are as follows:
fud-L-up:5'-TTACGAATTCGAGCTCGGTAAAGCCACCGGATTTAGCGC-3'; SEQ ID NO. 37;
fud-L-down: 5'-CCCAGTTAGCCTACCGAAAAATCAA-3'; SEQ ID NO. 38;
fud-R-up: 5'-TTCGGTAGGCTAACTGGGCCCTGGCTTTGGAGAGTTACT-3'; SEQ ID NO. 39;
fud-R-down: 5'-TCGACTCTAGAACACGATTCTCAAGCTTACTGCG-3'; SEQ ID NO. 40.

The plasmid vector PK-JL is linearized with primers pk-fud-F and pk-fud-R to obtain a pk-fud vector, and the primer sequences are as follows:
pk-fud-F: 5'-AGAATCGTGTTCTAGAGTCGACCTGCAGGC-3'; SEQ ID NO. 41;
pk-fud -R: 5'-TTTACCGAGCTCGAATTCGTAATCATGT-3'; SEQ ID NO. 42.

The gene amplification system and the gene amplification procedure are the same as above, and fud-L, fud-R and pk-fud vectors are obtained; the verification method of ligation and transformation is the same as above, and *Escherichia coli* WJ004 is obtained; and after propagation and plasmid extraction, WJ004 plasmid is obtained.

The *Escherichia coli* WJ004 plasmid is electrotransformed into competence of *Corynebacterium glutamicum* CG003. The competence preparation, electrotransformation, sacB verification, and culture with sucrose culture medium are the same as above, and the fud verification (the size band is 3431 bp) primers are as follows:
fud-verification-F: 5'-CTTTCTTGGGAAAGGCCCG-3'; SEQ ID NO. 43;
fud-verification-R: 5'-TGGCACCTACAACCTCACCAA-3'; SEQ ID NO. 44.

The bacteria that are verified with verification primers to succeed in knock-out are inoculated in LBHIS liquid medium for culture, and then streaked on LBHIS solid medium plate for purification and re-verification; and after confirmation, the bacteria can be preserved for use, and cgATCC13032Δ*pcaHG*Δ*van*Δ*vdh*Δ*fud* is named *Corynebacterium glutamicum* CG004.

(5) With genome DNA of *Escherichia coli* k12 as a template, a fragment *tktA* (Gene ID: 947420) is amplified with primers tktA-up/tktA-down to obtain the *tktA* fragment derived from *Escherichia coli* with a seamless coloning homologous arm and a ribosome bind site (RBS) ligated with aroG.

A base A on a 436^{th} site of an *aroG* gene sequence derived from *Escherichia coli* k12 is mutated to a base G, and a feedback-resistant suppressor gene sequence (as shown by SEQ ID NO. 45) is obtained; and with feedback-resistant suppressor gene sequence as a template, the fragment *aroG* is amplified with primers aroG-up/aroG-down to obtain an *aroG* fragment derived from the *Escherichia coli* with a seamless coloning homologous arm and RBS ligated with plasmid.

With a codon-optimized *comt* fragment (as shown by SEQ ID NO. 46) of *Rattus norvegicus* synthesized by Beijing Genomics Institute as a template, the fragment *comt* is amplified with primers comt-up/comt-down to obtain the *comt* fragment derived from *Rattus norvegicus* with a seamless coloning homologous arm and RBS ligated with *tktA.*

With a codon-optimized *car* gene fragment of *Nocardia iowensis* synthesized by Beijing Genomics Institute (as shown by SEQ ID NO. 47) as a template, the fragment *car* is amplified with primers car-up/car-down to obtain the *car* fragment derived from *Nocardia iowensis* with a seamless coloning homologous arm and RBS ligated with *metX.*

With a codon-optimized *sfp* fragment (as shown by SEQ ID NO. 48) of *Mycobacterium marinum* synthesized by Beijing Genomics Institute as a template, the fragment *sfp* is amplified with primers sfp-up/sfp-down to obtain the *sfp* fragment derived from *Mycobacterium marinum* with a seamless coloning homologous arm and RBS ligated with *car* and plasmid.

With the genome DNA of *Corynebacterium glutamicum* ATCC13032 as a template, the fragment *metE* (as shown by SEQ ID NO. 49) is amplified with primers metE-up/metE-down to obtain a *metE* fragment derived from *Corynebacterium glutamicum* with a seamless coloning homologous arm and RBS ligated with *ahcY* and plasmid.

With the genome DNA of *Corynebacterium glutamicum* ATCC13032 as a template, the fragment *metK* (as shown by SEQ ID NO. 50) is amplified with primers metK-up/metK-down to obtain a *metK* fragment derived from *Corynebacterium glutamicum* with a seamless coloning homologous arm and RBS ligated with plasmid.

With the genome DNA of *Corynebacterium glutamicum* ATCC13032 as a template, the fragment *metX* (as shown by SEQ ID NO. 51) is amplified with primers metX-up/metX-down to obtain a *metX* fragment derived from *Corynebacterium glutamicum* with a seamless coloning homologous arm and RBS ligated with *metK.*

With the genome DNA of *Corynebacterium glutamicum* ATCC13032 as a template, the fragment *ahcY* (as shown by SEQ ID NO. 52) is amplified with primers ahcY-up/ahcY-down to obtain an *ahcY* fragment derived from *Corynebacterium glutamicum* with a seamless coloning homologous arm and RBS ligated with comt.

The primer sequences are as follows:
tktA-up:5'-TAAAAGCGCGTCGCGGGTAAAAGGAGGATATACATATGTCCT CACGTAAAGAGCTTGC-3'; SEQ ID NO. 53;
tktA-down: 5'-TTACAGCAGTTCTTTTGCTTTCGC-3'; SEQ ID NO. 54;
aroG-up:5'-GGAAACAGACCATGGAATTCAAGGAGGATATACATATGAAT TATCAGAACGACGATTTACGC-3'; SEQ ID NO. 55;
aroG-down: 5'-TTACCCGCGACGCGCTTTTA-3'; SEQ ID NO. 56;
comt-up:5'-AAAGCAAAAGAACTGCTGTAAAAGGAGGATATACATATGGG TGATACCAAAGAACAGCG-3'; SEQ ID NO. 57;
comt-down: 5'-TTAAGATTTATCAGGGCTACTAGGTCCC-3'; SEQ ID NO. 58;
car-up:5'-CATCGAGTTCTACATCTAATCTAGAAAGGAGGATATACATATG GCAGTGGATTCCCCAG-3'; SEQ ID NO. 59;
car-down: 5'-TTACAGCAGCTGCAGCAGTTC-3'; SEQ ID NO. 60;
sfp-up:5'-AACTGCTGCAGCTGCTGTAAGGATCCCCAAGGAGGATATACAT ATGACCGTGGGCACTCTG-3'; SEQ ID NO. 61;
sfp-down:5'-CGCCAAAACAGCCAAGCTGAATTCGAGCTCGGTACCCTTAC AGCACGATTGCAGTCAG-3'; SEQ ID NO. 62;
metE-up:5'-CGGAGCACTACCGCTACTAAAAGGAGGATATACATATGACT TCCAACTTTTCTTCCACTG-3'; SEQ ID NO. 63;
metE-down:5'-TCTAGAGGATCCCCGGGTACCGAGCTCTTAGATAGTTGCT CCGATTTTCTCACG-3'; SEQ ID NO. 64;
metK-up:5'-TTAAGCTTGCATGCCTGCAGGTCGACAAGGAGGATATACAT GTGGCTCAGCCAACCG-3'; SEQ ID NO. 65;
metK-down: 5'-TTAGGCCAACTTGAGGGCTG-3'; SEQ ID NO. 66;
metX-up:5'-CAGCCCTCAAGTTGGCCTAAAAGGAGGATATACATATGCCC ACCCTCGCG-3'; SEQ ID NO. 67;
metX-down: 5'-TCTAGATTAGATGTAGAACTCGATGTAGGTCG-3'; SEQ ID NO. 68;
ahcY-up:5'-CCTAGTAGCCCTGATAAATCTTAAAAGGAGGATATACATATG GACTTCAAGGTTGCCGA-3'; SEQ ID NO. 69;
ahcY-down: 5'-TTAGTAGCGGTAGTGCTCCG-3'; SEQ ID NO. 70.

The gene amplification system and the gene amplification procedure are the same as above.

PCR products are subjected to agarose gel electrophoresis and product recovery to obtain corresponding gene products from different sources, such as *tktA*, *aroG*, *comt*, *car*, *sfp*, *metE*, *metK*, *metX* and *ahcY.*

The plasmid vector pEC-XK99E is linearized with the primers pec-F and pec-R to obtain a pec vector, and the primer sequences are as follows:
pec-F: 5'-TACCCGGGGATCCTCTAGAGTC-3'; SEQ ID NO. 71;
pec-R: 5'-GAATTCCATGGTCTGTTTCCTGTG-3'; SEQ ID NO. 72.

The amplification system and procedure is the same as the gene amplification system and procedure.

The plasmid vector pXMJ19 is linearized with the primers px-F and px-R to obtain a pxmj vector, and the primer sequences are as follows:
px-F: 5'-CGAATTCAGCTTGGCTGTTTTGGCG-3'; SEQ ID NO. 73;
px-R: 5'-TGCAGGCATGCAAGCTTAATTAATT-3'; SEQ ID NO. 74.

1.5 µl of *tktA* vector, 2.5 µl of *aroG* vector, 1.0 µl of *comt* vector, 1.5 µl of *metE* vector, 1.5 µl of *ahcY* vector, and 2.0 µl of pec vector from different sources, and 10.0 µl of Gibson enzyme are added into a PCR tube.

The ligation temperature is 50°C, the ligation time is 15 min, and a whole system is 20 µL.

20 µl of total ligation system of Gibson ligation is transformed into *E.coli* Trans10 commercial competence for culture and preservation. The transformation operation is strictly carried out according to instructions; after cultured at 37°C for 1 h, the whole system is coated on an LB plate (containing 50µg/mL kanamycin), then the whole system is cultured at 37°C for 12 h, and about 10 to 20 single colonies are selected for colony PCR amplification and DNA sequencing verification. Primers for colony PCR amplification and DNA sequencing are as follows:
Pec-F1: 5'-GGCTGTGCAGGTCGTAAATCAC-3'; SEQ ID NO. 75;
Pec-R1: 5'-AGTTCCCTACTCTCGCATGGG-3'; SEQ ID NO. 76.

A correct single colony is selected, and named *Escherichia coli* WJ101; and after propagation and plasmid extraction, WJ101 plasmid is obtained.

3.0 µl of *car* vector, 1.0 µl of *sfp* vector, 2.0 µl of *metK* vector, 2.0 µl of *metX vector*, 2.0 µl of pxmj vector, and 10.0 µl of Gibson enzyme are added into a PCR tube.

The ligation temperature is 50°C, the ligation time is 15 min, and a whole system is 20 µL.

20 µl of total ligation system of Gibson ligation is transformed into *E.coli* Trans10 commercial competence (TransGen Biotech) for culture and reservation. The transformation operation is strictly carried out according to instructions; after cultured at 37°C for 1 h, the whole system is coated on an LB plate (containing 50µg/mL kanamycin), then the whole system is cultured at 37°C for 12 h, and about 10 to 20 single colonies are selected for colony PCR amplification and DNA sequencing verification. Primers for colony PCR amplification and DNA sequencing are as follows:
Pxmj-F: 5'-CTGTGGTATGGCTGTGCAGGTC-3'; SEQ ID NO. 77;
Pxmj-R: 5'-ATGCCTGGCAGTTCCCTACT-3'; SEQ ID NO. 78.

A correct single colony is selected, and named *Escherichia coli* WJ201; and after propagation and plasmid extraction, WJ201 plasmid is obtained.

2.0 µl of *Escherichia coli* WJ101 plasmid and 2.0 µl of *Escherichia coli* WJ201 plasmid are electrotransformed into competence of *Corynebacterium glutamicum* CG004 at the same time. The competence preparation and electrotransformation are the same as above. The plasmid is uniformly coated on the LBHIS solid culture medium plate containing 50 µg/mL kanamycin and 5 µg/mL chloramphenicol and cultured at 30°C for 24-36 h.

The single colony growing on the plate is selected to verify kana and *cm* genes, and only the colony that can grow on a kana and chloramphenicol resistant plate and also can amplify kana and cm genes is a target strain, which is named *Corynebacterium glutamicum* CG101, and the verification primers are as follows:
kana-F: 5'-ATGATTGAACAAGATGGATTGCACG-3'; SEQ ID NO. 79;
kana-R: 5'-TCAGAAGAACTCGTCAAGAAGGC-3'; SEQ ID NO. 80;
Cm-R: 5'-CCTGCCACTCATCGCAGTAC-3'; SEQ ID NO. 81;
Cm-F: 5'-ATGGAGAAAAAAATCACTGGATATACCACC-3'; SEQ ID NO. 82.

The *Corynebacterium glutamicum* CG101, a genetically engineered strain of *Corynebacterium glutamicum,* is subjected to 50 mL system fermentation verification, an LBG culture medium is used for culturing seeds, and fermentation culture medium solution is used for fermentation.

The fermentation of the *Corynebacterium glutamicum* CG101 includes three steps:

Test tube inoculation: 100 µL of *Corynebacterium glutamicum* CG101 that is preserved at the temperature of -80°C is collected and transferred into a test tube containing 4 mL of LBHIS liquid culture (containing 50 µg/mL kanamycin and 5 µg/mL chloramphenicol) and cultured at 30°C for 12 h-14 h.

Seed inoculation: the test tube after cultured for 12-14 h is taken out, 1 mL of bacterial solution in the test tube is transferred into the LBG liquid culture medium of a 20 mL system (a 100 mL baffle-free conical bottle is used as a container, which contains 50 µg/mL kanamycin and 5 µg/mL chloramphenicol), and cultivated in a constant temperature shaker at 30°C and 200 rpm for 12-14 h.

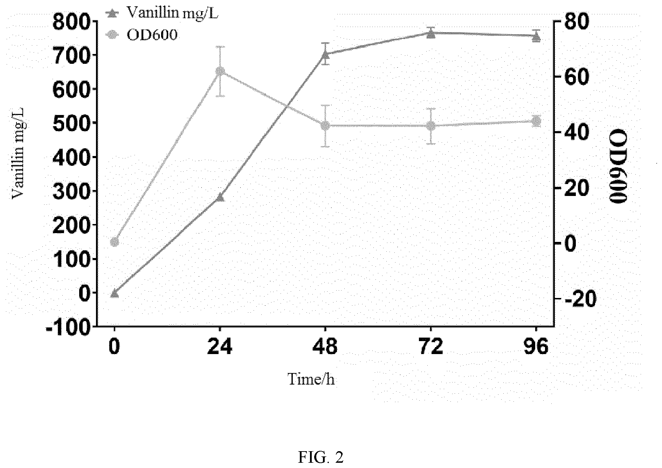

Inoculation of fermentation culture medium: a seed weight inoculated in the fermentation solution of 50 mL system (250 mL conical bottle with a baffle is used as a container, and the fermentation culture medium contains 50 µg/mL kanamycin and 5 µg/mL chloramphenicol) is 5% of the total fermentation system, the pH of the fermentation solution is regulated by strong ammonia water, the fermentation solution is cultured in the constant temperature shaker at 30°C and 200 rpm for 96 h (0.8 mM IPTG is added for induction after culture for 3 h), every 24 h is a sampling point in the fermentation process, and OD₆₀₀ and a vanillin concentration of a target product are detected. (1) Detection of OD₆₀₀: the fermentation solution is charged into a cuvette, and diluted 50 times with deionized water, and then a light absorption value (600 nm) is measured by an ultraviolet spectrophotometer, and the OD₆₀₀ is determined. (2) Determination of vanillin concentration in the fermentation solution: the vanillin in the fermentation solution is detected by a UltiMate 3000 high performance liquid chromatograph (HPLC, Thermo Fisher Scientific); and the detection method includes: a flow phase includes a phase A (0.1% formic acid aqueous solution) and a phase B (pure methanol); ZORBAX SB-C18 reversed column is used as the chromatographic column, a column temperature is set at 28°C, a flow rate of the flow phase is set at 1.0 mL/min, an ultraviolet detector is used for detection, and an ultraviolet absorption wavelength is set at 260 nm; and finally, contents of products and other metabolites are calculated from data peaks of different fermentation samples according to the standard curves drawn by standard samples. Results are shown in Table 1 and Fig. 2.

**Table 1**

| Time (h) | Vanillin (mg/L) | OD₆₀₀ |
|---|---|---|
| 0 | 0 | 0.464 |
| 24 | 282.53 | 61.9 |
| 48 | 702.82 | 42.3 |
| 72 | 765.85 | 42.2 |
| 96 | 756.87 | 43.9 |

### Embodiment 2

A method for constructing a genetically engineered bacterium using glucose as a substrate for de novo synthesis of vanillin includes the following steps:
With the genome DNA of *Corynebacterium glutamicum* ATCC13032 as a template, a fragment *tktA* (as shown by SEQ ID NO. 83) is amplified with primers tktA-up2/tktA-down2 obtain a *tktA* fragment derived from *Corynebacterium glutamicum* with a seamless coloning homologous arm and ribosome bind site (RBS) ligated with *aroG.*

With the genome DNA of *Corynebacterium glutamicum* ATCC13032 as a template, a fragment *aroG* (as shown by SEQ ID NO. 84) is amplified with primers aroG-up2/aroG-down2 to obtain an *aroG* fragment derived from *Corynebacterium glutamicum* with a seamless coloning homologous arm and RBS ligated with plasmid.

With a codon-optimized *comt* fragment (as shown by SEQ ID NO. 85) of *Arabidopsis thaliana* synthesized by Beijing Genomics Institute as a template, the fragment *comt* is amplified with primers comt-up2/comt-down2 to obtain the *comt* fragment derived from *Arabidopsis thaliana* with a seamless coloning homologous arm and RBS ligated with *tktA.*

With a codon-optimized *car* gene fragment (as shown by SEQ ID NO. 86) of *Mycobacterium marinum* synthesized by Beijing Genomics Institute as a template, the fragment *car* is amplified with primers car-up2/car-down2 to obtain the *car* fragment derived from *Mycobacterium marinum* with a seamless coloning homologous arm and RBS ligated with *met2.*

With a codon-optimized *sfp* fragment (as shown by SEQ ID NO. 87) of *Nocardia iowensis* synthesized by Beijing Genomics Institute as a template, the fragment *sfp* is amplified with primers sfp-up2/sfp-down2 to obtain the *sfp* fragment drived from *Nocardia iowensis* with a seamless coloning homologous arm and RBS ligated with *car* and plasmid.

With the genome DNA of *Escherichia coli* k12 as a template, a fragment *metE* (Gene ID: 948323) is amplified with primers met E-up2/metE-down2 to obtain the *metE* fragment derived from *Escherichia coli* with a seamless coloning homologous arm and RBS ligated with *ahcY* and plasmid.

With the genome DNA of *Escherichia coli* k12 as a template, a fragment *metK* (Gene ID: 945389) is amplified with primers metK-up2/metK-down2 to obtain the *metK* fragment derived from *Escherichia coli* with a seamless coloning homologous arm and RBS ligated with plasmid.

With the genome DNA of *Saccharomyces cerevisiae* S288C as a template, a fragment *met2* (Gene ID: 855444) is amplified with primers met2-up2/met2-down2 to obtain the *met2* fragment derived from *Saccharomyces cerevisiae* S288C with a seamless coloning homologous arm and RBS ligated with *metK.*

With the genome DNA of *Corynebacterium glutamicum* ATCC13032 as a template, the fragment *ahcY* (as shown by SEQ ID NO. 52) is amplified with primers ahcY-up2/ahcY-down2 to obtain an *ahcY* fragment derived from *Corynebacterium glutamicum* with a seamless coloning homologous arm and RBS ligated with *comt.*

The primer sequences are as follows:
tktA-up2:5'-ACGCCGAGCAGCAGCCAAGTAAAAGGAGGATATACATGTGG ACACCAAGGCTGTAGA-3'; SEQ ID NO. 88;
tktA-down2: 5'-TTAACCGTTAATGGAGTCCTTGG-3'; SEQ ID NO. 89;
aroG-up2:5'-GGAAACAGACCATGGAATTCAAGGAGGATATACATATGAG TTCTCCAGTCTCACTCG-3'; SEQ ID NO. 90;
aroG-down2: 5'-TTACTTGGCTGCTGCTCG-3'; SEQ ID NO. 91;
comt-up2:5'-CCAAGGACTCCATTAACGGTTAAAAGGAGGATATACATATG GGTAGCACCGCGG-3'; SEQ ID NO. 92;
comt-down2: 5'-TTACAGTTTTTTCAGCAGTTCAATCAGG-3'; SEQ ID NO. 93;
car-up2:5'-AAGAAGTTACCAACTGGTAGtctagaAAGGAGGATATACATATG TCCCCAATCACCCGC-3'; SEQ ID NO. 94;
Car-down2: 5'-TTGGATCCTTACAGCAGGCCCAGCAGG-3'; SEQ ID NO. 95;
sfp-up2:5'-CTGCTGGGCCTGCTGTAAggatccAAGGAGGATATACATATGAT CGAAACCATCCTG-3'; SEQ ID NO. 96;
sfp-down2:5'-CGCCAAAACAGCCAAGCTGAATTCGAGCTCGGTACCCTTA TGCGTATGCGATTGCGGTC-3'; SEQ ID NO. 97;
metE-up2:5'-CGGAGCACTACCGCTACTAAAAGGAGGATATACATATGAC AATATTGAATCACACCCTCG-3'; SEQ ID NO. 98;
metE-down2:5'-GACTCTAGAGGATCCCCGGGTACCGAGCTCTTACCCCCG ACGCAAGTTC-3'; SEQ ID NO. 99;
metK-up2:5'-TTAATTAAGCTTGCATGCCTGCAGGTCGACAAGGAGGATAT ACATATGGCAAAACACCTTTTTACGTC-3'; SEQ ID NO. 100;
metK-down2: 5'-TTACTTCAGACCGGCAGCA-3'; SEQ ID NO. 101;
met2-up2:5'-GCTGCCGGTCTGAAGTAAAAGGAGGATATACATATGTCGCA TACTTTAAAATCGAAAACG-3'; SEQ ID NO. 102;
met2-down2: 5'-CTACCAGTTGGTAACTTCTTCGG-3'; SEQ ID NO. 103;
ahcY-up2:5'-ATTGAACTGCTGAAAAAACTGTAAAAGGAGGATATACATAT GGACTTCAAGGTTGCCGA-3'; SEQ ID NO. 104;
ahcY-down2: same as *ahcY-*down.

The gene amplification system and the gene amplification procedure are the same as above.

PCR products are subjected to agarose gel electrophoresis and product recovery to obtain corresponding gene products from different sources, such as *tkt*, *aroG*, *comt*, *car*, *sfp*, *metE*, *metK*, *metX* and *ahcY.*

The plasmid vector pEC-XK99E is linearized with the primers pec-F and pec-R to obtain a pec vector, the same as embodiment 1.

The ligation system, procedure, transformation and verification are the same as Embodiment 1. A correct single colony is selected, and named *Escherichia coli* WJ102; and after propagation and plasmid extraction, WJ102 plasmid is obtained.

The plasmid vector pXMJ19 is linearized with the primers px-F and px-R to obtain a pxmj vector, the same as embodiment 1.

The ligation system, procedure, transformation and verification are the same as embodiment 1. A correct single colony is selected, and named *Escherichia coli* WJ202; and after propagation and plasmid extraction, WJ202 plasmid is obtained.

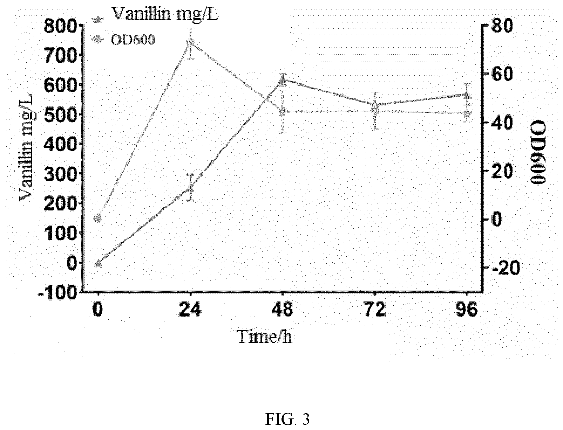

The *Escherichia coli* WJ102 plasmid and the *Escherichia coli* WJ202 plasmid are electrotransformed into competence of *Corynebacterium glutamicum* CG004 at the same time (prepared in a same way as embodiment 1), and named *Corynebacterium glutamicum* CG102. The competence preparation, electrotransformation, verification and fermentation are the same as embodiment 1. Results are shown in Table 2 and Fig. 3.

**Table 2**

| Time (h) | Vanillin (mg/L) | OD₆₀₀ |
|---|---|---|
| 0 | 0 | 0.468 |
| 24 | 252.87 | 72.8 |
| 48 | 616.60 | 44.4 |
| 72 | 531.45 | 44.7 |
| 96 | 566.96 | 43.7 |

### Embodiment 3

A method for constructing a genetically engineered bacterium using glucose as a substrate for de novo synthesis of vanillin includes the following steps:
With the genome DNA of *Saccharomyces cerevisiae* S288C as a template, a fragment *tk11* (Gene ID: 856188) is amplified with primers tkl1up3/ tkl1-down3 to obtain *atkl1* fragment derived from *Saccharomyces cerevisiae* S288C with a seamless coloning homologous arm and RBS ligated with *aro4.*

With the genome DNA of *Saccharomyces cerevisiae* S288C as a template, a fragment *aro4* (Gene ID: 852551) is amplified with primers aro4-up3/aro4-down3 to obtain a *aro4* fragment derived from *Saccharomyces cerevisiae* S288C with a seamless coloning homologous arm and RBS ligated with plasmid.

With a codon-optimized *comt* fragment (as shown by SEQ ID NO.105) of *Homo sapiens* synthesized by Beijing Genomics Institute as a template, the fragment *comt* is amplified with primers comt-up3/comt-down3 to obtain a *comt* fragment derived from *Homo sapiens* with a seamless coloning homologous arm and RBS ligated with *tkl1.*

With a codon-optimized *car* gene fragment (as shown by SEQ ID NO.47) of *Nocardia iowensis* synthesized by Beijing Genomics Institute as a template, the fragment *car* is amplified with primers car-up3/car-down3 to obtain a *car* fragment derived from *Nocardia iowensis* with a seamless coloning homologous arm and RBS ligated with *met2.*

With a codon-optimized *sfp* gene fragment (as shown by SEQ ID NO. 106) of *Bacillus subtilis* synthesized by Beijing Genomics Institute as a template, the fragment *sfp* is amplified with primers sfp-up3/sfp-down3 to obtain a *sfp* fragment derived from *Bacillus subtilis* with a seamless coloning homologous arm and RBS ligated with *car* and plasmid.

With the genome DNA of *Saccharomyces cerevisiae* S288C as a template, a fragment *met6* (Gene ID: 856825) is amplified with primers met6-up3/met6-down3 to obtain a *met6* fragment derived from *Saccharomyces cerevisiae* S288C with a seamless coloning homologous arm and RBS ligated with *ahcY* and plasmid.

With the genome DNA of *Saccharomyces cerevisiae* S288C as a template, the fragment *SAM2* (Gene ID: 852113) is amplified with primers SAM2-up3/ SAM2-down3 to obtain a *SAM2* fragment derived from *Saccharomyces cerevisiae* S288C with a seamless coloning homologous arm and RBS ligated with plasmid.

With the genome DNA of *Saccharomyces cerevisiae* S288C as a template, the fragment *met2* (Gene ID: 855444) is amplified with primers met2-up3/met2-down3 to obtain a *met2* fragment derived from *Saccharomyces cerevisiae* S288C with a seamless coloning homologous arm and RBS ligated with *SAM2.*

With the genome DNA of *Corynebacterium glutamicum* ATCC13032 as a template, the fragment *ahcY* (as shown by SEQ ID NO. 52) is amplified with primers ahcY-up3/ahcY-down3 to obtain an *ahcY* fragment derived from *Corynebacterium glutamicum* with a seamless coloning homologous arm and RBS ligated with *comt.*

The primer sequences are as follows:
tkl1up3:5'-AGAGAAGTTAACAAGAAATAGAAGGAGGATATACATATGAC TCAATTCACTGACATTGATAAGC-3'; SEQ ID NO. 107;
tkl1-down3: 5'-TTAGAAAGCTTTTTTCAAAGGAGAAATTAGC-3'; SEQ ID NO. 108;
aro4-up3:5'-GGAAACAGACCATGGAATTCAAGGAGGATATACATATGAGT GAATCTCCAATGTTCGC-3'; SEQ ID NO. 109;
aro4-down3: 5'-CTATTTCTTGTTAACTTCTCTTCTTTGTCTG-3'; SEQ ID NO. 110;
comt-up3:5'-CTCCTTTGAAAAAAGCTTTCTAAAAGGAGGATATACATATG CCGGAAGCCCCG-3'; SEQ ID NO. 111;
comt-down3: 5'-TTACGGACCCGCTTCACTACC-3'; SEQ ID NO. 112;
car-up3:5'-AAGAAGTTACCAACTGGTAGTCTAGAAAGGAGGATATACATA TGGCAGTGGATTCCCCAG-3'; SEQ ID NO. 113;
car-down3: same as car-down;
sfp-up3:5'-CTGCTGCAGCTGCTGTAAGGATCCCCAAGGAGGATATACATA TGAAGATCTACGGCATC-3'; SEQ ID NO. 114;
sfp-down3:5'-CTGAATTCGAGCTCGGTACCCTTACAGCAGTTCTTCGTAGG ACAC-3'; SEQ ID NO. 115;
met6-up3:5'-CGGAGCACTACCGCTACTAAAAGGAGGATATACATATGGTT CAATCTGCTGTCTTAGG-3'; SEQ ID NO. 116;
met6-down3:5'-TCTAGAGGATCCCCGGGTACCGAGCTCTTAATTCTTGTAT TGTTCACGGAAGTACTTG-3'; SEQ ID NO. 117;
SAM2-up3:5'-TTAAGCTTGCATGCCTGCAGGTCGACAAGGAGGATATACA TATGTCCAAGAGCAAAACTTTCTTAT-3'; SEQ ID NO. 118;
SAM2-down3: 5'-TTAAAATTCCAATTTCTTTGGTTTTTCCC-3'; SEQ ID NO. 119;
met2-up3:5'-CCAAAGAAATTGGAATTTTAAAAGGAGGATATACATATGTC GCATACTTTAAAATCGAAAACG-3'; SEQ ID NO. 120;
met2-down3: same as met2-down2;
ahcY-up3:5'-GGTAGTGAAGCGGGTCCGTAAAAGGAGGATATACATATGG ACTTCAAGGTTGCCGA-3'; SEQ ID NO. 121;
ahcY-down3: same as ahcY-down.

The gene amplification system and the gene amplification procedure are the same as above.

PCR products are subjected to agarose gel electrophoresis and product recovery to obtain corresponding gene products from different sources, such as tkl1, aro4, *comt*, *car*, *sfp*, *met6*, *SAM2*, *met2* and *ahcY.*

The plasmid vector pEC-XK99E is linearized with the primers pec-F and pec-R to obtain a pec vector, the same as embodiment 1.

The ligation system, procedure, transformation and verification are the same as embodiment 1. A correct single colony is selected, and named *Escherichia coli* WJ103; and after propagation and plasmid extraction, WJ103 plasmid is obtained.

The plasmid vector pXMJ19 is linearized with the primers px-F and px-R to obtain a pxmj vector, the same as embodiment 1.

The ligation system, procedure, transformation and verification are the same as embodiment 1. A correct single colony is selected, and named *Escherichia coli* WJ203; and after propagation and plasmid extraction, WJ203 plasmid is obtained.

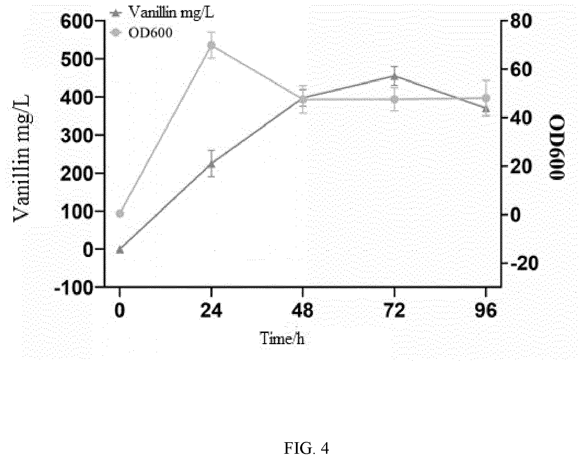

The *Escherichia coli* WJ103 plasmid and the *Escherichia coli* WJ203 plasmid are electrotransformed into competence of *Corynebacterium glutamicum* CG004 at the same time (prepared in a same way as Embodiment 1), and named *Corynebacterium glutamicum* CG103. The competence preparation, electrotransformation, verification and fermentation are the same as Embodiment 1. Results are shown in Table 3 and Fig. 4.

**Table 3**

| Time (h) | Vanillin (mg/L) | OD₆₀₀ |
|---|---|---|
| 0 | 0 | 0.466 |
| 24 | 224.83 | 69.9 |
| 48 | 397.38 | 47.5 |
| 72 | 454.82 | 47.6 |
| 96 | 370.38 | 48.1 |

### Embodiment 4

A method for constructing a genetically engineered bacterium using glucose as a substrate for de novo synthesis of vanillin includes the following steps:
With the genome DNA of *Bacillus subtilis* 168 as a template, a fragment *tktA* (Gene ID: 937377) is amplified with primers tktA-up4/tktA-down4 to obtain a *tktA* fragment derived from *Bacillus subtilis* 168 with a seamless coloning homologous arm and ribosome bind site (RBS) ligated with *aroX.*

With the genome DNA of *Bacillus subtilis168* as a template, a fragment *aroX* (Gene ID: 937853) is amplified with primers aroX-up4/aroX-down4 to obtain an *aroX* fragment derived from *Bacillus subtilis* 168 with a seamless coloning homologous arm and RBS ligated with plasmid.

With a codon-optimized *comt* fragment (as shown by SEQ ID NO. 122) of *Coffea canephora (Robusta coffee)* synthesized by Beijing Genomics Institute as a template, the fragment *comt* is amplified with primers comt-up4/comt-down4 to obtain a *comt* fragment derived from *Coffea canephora* with a seamless coloning homologous arm and RBS ligated with *tktA.*

With a codon-optimized *car* gene fragment (as shown by SEQ ID NO. 47) of *Nocardia iowensis* synthesized by Beijing Genomics Institute as a template, the fragment *car* is amplified with primers car-up4/car-down4 to obtain a *car* fragment derived from *Nocardia iowensis* with a seamless coloning homologous arm and RBS ligated with *metAA.*

With a codon-optimized *sfp* fragment (as shown in SEQ ID NO. 48) of *Mycobacterium marinum* synthesized by Beijing Genomics Institute as a template, the fragment *sfp* is amplified with primers sfp-up4/sfp-down4 to obtain a *sfp* fragment derived from *Mycobacterium marinum* with a seamless coloning homologous arm and RBS ligated with *car* and plasmid.

With the genome DNA of *Bacillus subtilis168* as a template, the fragment *metE* (Gene ID: 936480) is amplified with primers metE-up4/metE-down4 to obtain a *metE* fragment derived from *Bacillus subtilis 168* with a seamless coloning homologous arm and RBS ligated with *ahcY* and plasmid.

With the genome DNA of *Bacillus subtilis168* as a template, the fragment *metK* (Gene ID: 937090) is amplified with primers metK-up4/metK-down4 to obtain a *metK* fragment derived from *Bacillus subtilis 168* with a seamless coloning homologous arm and RBS ligated with plasmid.

With the genome DNA of *Bacillus subtilis168* as a template, the fragment *metAA* (Gene ID: 939083) is amplified with primers metAA-up4/metAA-down4 to obtain a *metAA* fragment derived from *Bacillus subtilis* 168 with a seamless coloning homologous arm and RBS ligated with *metK.*

With the genome DNA of *Corynebacterium glutamicum* ATCC13032 as a template, the fragment *ahcY* (as shown by SEQ ID NO. 52) is amplified with primers ahcY-up4/ahcY-down4 to obtain a *ahcY* fragment derived from *Corynebacterium glutamicum* with a seamless coloning homologous arm and RBS ligated with *comt.*

The primer sequences are as follows:
tktA-up4:5'-CAATGGTGAAAGTCAACGCTTAAAAGGAGGATATACATATG GATACAATTGAAAAGAAATCAGTTGC-3'; SEQ ID NO. 123;
tktA-down4: 5'-TTACTTATTGATTAATGCCTTAACTCGATTC-3'; SEQ ID NO. 124;
aroX-up4:5'-GGAAACAGACCATGGAATTCAAGGAGGATATACATATGAG CAACACAGAGTTAGAGC-3'; SEQ ID NO. 125;
aroX-down4: 5'-TTAAGCGTTGACTTTCACCATTG-3'; SEQ ID NO. 126;
comt-up4:5'-AGGCATTAATCAATAAGTAAAAGGAGGATATACATATGGCC GAAGAAGAAGCATG-3'; SEQ ID NO. 127;
comt-down4: 5'-TTATTTACACAGTTCCATAATCCAGGTATTC-3'; SEQ ID NO. 128;
car-up4: 
5’-TCCTTATGAATGGGACTAAtctagaAAGGAGGATATACATATGGCAGTGGATTCC
CCAG-3’; SEQ ID NO. 129;
car-down4: same as car-down1;
sfp-up4: same as sfp-up 1;
sfp-down4: same as sfp-down 1;
metE-up4:5'-CGGAGCACTACCGCTACTAAAAGGAGGATATACATATGAC AACCATCAAAACATCGAAT-3'; SEQ ID NO. 130;
metE-down4:
   5'-TCTAGAGGATCCCCGGGTACCGAGCTCTTATACTAGCTGTGTCTGCTGTGC-3'; SEQ ID NO. 131;
metK-up4:5'-TTAAGCTTGCATGCCTGCAGGTCGACAAGGAGGATATACAT ATGAGTAAAAATCGTCGTTTATTTACATC-3'; SEQ ID NO. 132;
metK-down4: 5'-TTATTCTCCTAACGCTTCTTTACGC-3'; SEQ ID NO. 133;
metAA-up4:5'-AAGAAGCGTTAGGAGAATAAAAGGAGGATATACATTTGC CTATTAATATACCAACACACCTG-3'; SEQ ID NO. 134;
metAA-down4: 5'-TTAGTCCCATTCATAAGGAGTTTCTTG-3'; SEQ ID NO. 135;
ahcY-up4: 
5'-TTATGGAACTGTGTAAATAAAAGGAGGATATACATATGGACTTCAAGGTTGCC
GA-3'; SEQ ID NO. 136;
ahcY-down4: same as ahcY-down.

The gene amplification system and the gene amplification procedure are the same as above.

PCR products are subjected to agarose gel electrophoresis and product recovery to obtain corresponding gene products from different sources, such as *tktA*, *aroX*, *comt*, *car*, *sfp*, *metE*, *metK*, *metAA* and *ahcY.*

The plasmid vector pEC-XK99E is linearized with the primers pec-F and pec-R to obtain a pec vector, the same as embodiment 1.

The ligation system, procedure, transformation and verification are the same as embodiment 1. A correct single colony is selected, and named *Escherichia coli* WJ104; and after propagation and plasmid extraction, WJ104 plasmid is obtained.

The plasmid vector pXMJ19 is linearized with the primers px-F and px-R to obtain a pxmj vector, the same as embodiment 1.

The ligation system, procedure, transformation and verification are the same as embodiment 1. A correct single colony is selected, and named *Escherichia coli* WJ204; and after propagation and plasmid extraction, WJ204 plasmid is obtained.

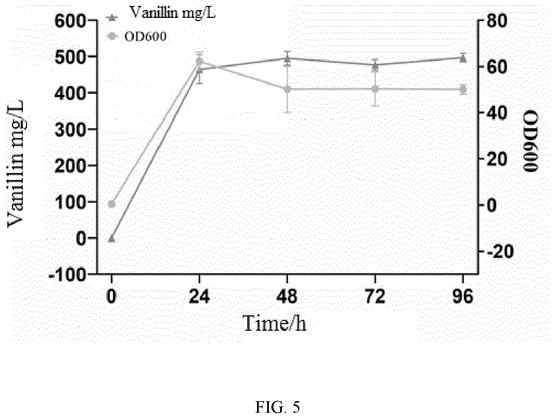

The *Escherichia coli* WJ104 plasmid and the *Escherichia coli* WJ204 plasmid are electrotransformed into competence of *Corynebacterium glutamicum* CG104 at the same time (prepared in a same way as embodiment 1), and named *Corynebacterium glutamicum* CG104. The competence preparation, electrotransformation, verification and fermentation are the same as embodiment 1. Results are shown in Table 4 and Fig. 5.

**Table 4**

| Time (h) | Vanillin (mg/L) | OD₆₀₀ |
|---|---|---|
| 0 | 0 | 0.464 |
| 24 | 464.72 | 61.7 |
| 48 | 495.19 | 52.1 |
| 72 | 477.08 | 50.6 |
| 96 | 497.08 | 49.9 |

### Embodiment 5

A method for constructing a genetically engineered bacterium using glucose as a substrate for de novo synthesis of vanillin includes the following steps:
With a codon-optimized *comt* fragment (as shown by SEQ ID NO. 137) of *Mus musculus* synthesized by Beijing Genomics Institute is as a template, the fragment *comt* is amplified with primers comt-up5/comt-down4 to obtain a *comt* fragment derived from *Mus musculus* with a seamless coloning homologous arm and RBS ligated with *tktA.*

The primer sequences are as follows:
comt-up5:5'-AAAGCAAAAGAACTGCTGTAAAAGGAGGATATACATATGC TGCTGGCGGCC-3'; SEQ ID NO. 138;
comt-down4: 5'-TTAGCTTTTAACCGGGCTGCT-3'; SEQ ID NO. 139;
ahcY-up5:5'-AGCAGCCCGGTTAAAAGCTAAAAGGAGGATATACATATGG ACTTCAAGGTTGCCGA-3'; SEQ ID NO. 140.

Other genes and primers are the same as embodiment 1.

The gene amplification system and the gene amplification procedure are the same as above.

PCR products are subjected to agarose gel electrophoresis and product recovery to obtain corresponding gene products from different sources, such as *tktA*, *aroG*, *comt*, *car*, *sfp*, *metE*, *metK*, *metX* and *ahcY.*

The plasmid vector pEC-XK99E is linearized with the primers pec-F and pec-R to obtain a pec vector, the same as embodiment 1.

The ligation system, procedure, transformation and verification are the same as embodiment 1. A correct single colony is selected, and named *Escherichia coli* WJ105; and after propagation and plasmid extraction, WJ105 plasmid is obtained.

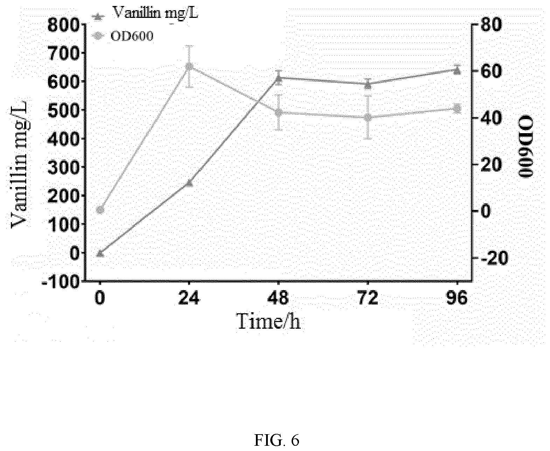

The *Escherichia coli* WJ105 plasmid and the *Escherichia coli* WJ201 plasmid are electrotransformed into competence of *Corynebacterium glutamicum* CG004 at the same time (prepared in a same way as embodiment 1), and named *Corynebacterium glutamicum* CG105. The competence preparation, electrotransformation, verification and fermentation are the same as embodiment 1. Results are shown in Table 5 and Fig. 6.

**Table 5**

| Time (h) | Vanillin (mg/L) | OD₆₀₀ |
|---|---|---|
| 0 | 0 | 0.470 |
| 24 | 246.97 | 60.53 |
| 48 | 601.81 | 43.19 |
| 72 | 590.10 | 42.18 |
| 96 | 633.39 | 44.27 |

The peak time of HPLC detection results of vanillin specimen is 17.231 min, the peak time of fermentation culture products of genetically engineered strains prepared in Embodiments 1-5 is 17.233 min, and the peak time of fermentation samples of engineered strains is the same as that of the vanillin specimen, which proves that the fermentation products of engineered strains are the target product-vanillin.

The above description of the disclosed embodiments enables those skilled in the art to implement or use the present invention. Various modifications to these embodiments are apparent for those skilled in the art. The general principles defined herein may be implemented in other embodiments without departing from the spirit or scope of the present invention. Therefore, the present invention will not be limited to these embodiments described herein, but shall conform to the widest scope consistent with the principles and novel characteristics disclosed herein.

## Claims

1. A genetically engineered bacterium using glucose as a substrate for de novo synthesis of vanillin, wherein
the genetically engineered bacterium is a recombinant *Corynebacterium glutamicum* modified by chassis microorganisms and comprising a vanillin synthesis module and a methyl cyclic regeneration module;
the modification of chassis microorganism is to take *Corynebacterium glutamicum* ATCC13032 as an original strain to knock out *pcaHG*, *van*, *vdh* and *fud* genes;
the vanillin synthesis module expresses a transketolase gene, a 3-deoxy-7-phosphoheptulonate synthase gene, an O-methyltransferase gene, a Carboxylic acid reductase gene and a 4'-phosphopantetheinyl transferase gene;
the methyl cyclic regeneration module expresses a 5-methyltetrahydropteroyltriglutamate-homocysteine methyltransferase gene, an S-adenosylmethionine synthase gene, a homoserine O-acetyltransferase gene and an adenosylhomocysteinase gene.

2. The genetically engineered bacterium using glucose as the substrate for de novo synthesis of vanillin according to claim 1, wherein
the transketolase gene comprises an endogenous *Corynebacterium glutamicum* transketolase gene or an exogenous transketolase gene;
the 3-deoxy-7-phosphoheptulonate synthase gene comprises an endogenous *Corynebacterium glutamicum*3-deoxy-7-phosphoheptulonate synthase gene or an exogenous 3-deoxy-7-phosphoheptulonate synthase gene;
the O-methyltransferase gene comprises an O-methyltransferase gene from *Arabidopsis thaliana (Mouseear cress)*, an O-methyltransferase gene from *Coffea canephora (Robusta coffee*), an O-methyltransferase gene from *Homo sapiens (Human*), an O-methyltransferase gene from *Rattus norvegicus (rat)*, and an O-methyltransferase gene from *Mus musculus (mouse)*;
the carboxylic acid reductase gene comprises a carboxylic acid reductase gene from *Nocardia iowensis* and a carboxylic acid reductase gene from *Mycobacterium marinum*; the 4'-phosphopantetheinyl transferase gene comprises a 4'-phosphopantetheinyl transferase gene from *Nocardia iowensis*, a 4'-phosphopantetheinyl transferase gene from *Mycobacterium marinum*, and a 4'-phosphopantetheinyl transferase gene from *Bacillus subtilis*;
the 5-methyltetrahydropteroyltriglutamate-homocysteine methyltransferase gene comprises an endogenous *Corynebacterium glutamicum* 5-methyltetrahydropteroyltriglutamate-homocysteine methyltransferase gene, or an exogenous 5-methyltetrahydropteroyltriglutamate-homocysteine methyltransferase gene;
the S-adenosylmethionine synthase gene comprises an endogenous *Corynebacterium glutamicum* S-adenosylmethionine synthase gene and an optional exogenous S-adenosylmethionine synthase gene;
the homoserine O-acetyltransferase gene comprises an endogenous *Corynebacterium glutamicum* homoserine O-acetyltransferase gene and an optional exogenous homoserine O-acetyltransferase gene;
the adenosylhomocysteinase gene comprises an endogenous *Corynebacterium glutamicum* adenosylhomocysteinase gene and an optional exogenous adenosylhomocysteinase gene; the nucleotide sequence of the endogenous *Corynebacterium glutamicum* transketolase gene is as shown by SEQ ID NO.83;
the nucleotide sequence of the endogenous *Corynebacterium glutamicum* 3-deoxy-7-phosphoheptulonate synthase gene is as shown by SEQ ID NO. 84;
the nucleotide sequence of the O-methyltransferase gene from *Arabidopsis thaliana* is as shown by SEQ ID NO. 85; the nucleotide sequence of the O-methyltransferase gene from *Coffea canephora* is as shown by SEQ ID NO. 122; the nucleotide sequence of the O-methyltransferase gene from *Homo sapiens* is as shown by SEQ ID NO. 105; the nucleotide sequence of the O-methyltransferase gene from *Rattus norvegicus* is as shown by SEQ ID NO. 46; and the nucleotide sequence of the O-methyltransferase gene from *Mus musculus* is as shown by SEQ ID NO. 137;
the nucleotide sequence of the carboxylic acid reductase gene from *Nocardia iowensis* is as shown by SEQ ID NO. 47; and the nucleotide sequence of the carboxylic acid reductase gene from *Mycobacterium marinum* is as shown by SEQ ID NO. 86;
the nucleotide sequence of the 4'-phosphopantetheinyl transferase gene from *Nocardia iowensis* is as shown by SEQ ID NO. 87; the nucleotide sequence of the 4'-phosphopantetheinyl transferase gene from *Mycobacterium marinum* is as shown by SEQ ID NO. 48; and the nucleotide sequence of the 4'-phosphopantetheinyl transferase gene from *Bacillus subtilis* is as shown by SEQ ID NO. 106;
the nucleotide sequence of the endogenous *Corynebacterium glutamicum* 5-methyltetrahydropteroyltriglutamate-homocysteine methyltransferase gene is as shown by SEQ ID NO. 49;
the nucleotide sequence of the endogenous *Corynebacterium glutamicum* S-adenosylmethionine synthase gene is as shown by SEQ ID NO. 50;
the nucleotide sequence of the endogenous *Corynebacterium glutamicum* homoserine O-acetyltransferase gene is as shown by SEQ ID NO. 51;
the nucleotide sequence of the endogenous *Corynebacterium glutamicum* adenosylhomocysteinase gene is as shown by SEQ ID NO. 52.

3. The genetically engineered bacterium using glucose as the substrate for de novo synthesis of vanillin according to claim 2, wherein
the exogenous transketolase gene is derived from *Escherichia coli* K12, *Saccharomyces cerevisiae* S288C or *Bacillus subtilis* 168; the transketolase gene derived from *Escherichia coli* K12: Gene ID: 947420; the transketolase gene derived from *Saccharomyces cerevisiae* S288C: Gene ID: 856188); the transketolase gene derived from *Bacillus subtilis* 168: Gene ID: 937377;
the exogenous 3-deoxy-7-phosphoheptulonate synthase gene is derived from *Escherichia coli* K12, *Saccharomyces cerevisiae* S288C or *Bacillus subtilis* 168; the nucleotide sequence of the 3-deoxy-7-phosphoheptulonate synthase gene derived from *Escherichia coli* K12 is as shown by SEQ ID NO. 45; the 3-deoxy-7-phosphoheptulonate synthase gene derived from *Saccharomyces cerevisiae* S288C: Gene ID: 852551; and the 3-deoxy-7-phosphoheptulonate synthase gene derived from *Bacillus subtilis* 168: Gene ID: 937853;
the exogenous 5-methyltetrahydropteroyltriglutamate-homocysteine methyltransferase gene is derived from *Escherichia coli* K12, *Saccharomyces cerevisiae* S288C or *Bacillus subtilis* 168; the 5-methyltetrahydropteroyltriglutamate-homocysteine methyltransferase gene derived from *Escherichia coli* K12: Gene ID: 948323; the 5-methyltetrahydropteroyltriglutamate-homocysteine methyltransferase gene derived from *Saccharomyces cerevisiae* S288C: Gene ID: 856825; and the 5-methyltetrahydropteroyltriglutamate-homocysteine methyltransferase gene derived from *Bacillus subtilis* 168: Gene ID: 936480;
the exogenous S-adenosylmethionine synthase gene is derived from *Escherichia coli* K12, *Saccharomyces cerevisiae* S288C or *Bacillus subtilis* 168; the S-adenosylmethionine synthase gene derived from *Escherichia coli* K12: Gene ID: 948323; the S-adenosylmethionine synthase gene derived from *Saccharomyces cerevisiae* S288C: Gene ID: 852113; and the S-adenosylmethionine synthase gene derived from *Bacillus subtilis* 168: Gene ID: 937090;
the exogenous homoserine O-acetyltransferase gene is derived from *Saccharomyces cerevisiae* S288C or *Bacillus subtilis* 168; the homoserine O-acetyltransferase gene derived from *Saccharomyces cerevisiae* S288C: Gene ID: 855444; and the homoserine O-acetyltransferase gene derived from *Bacillus subtilis* 168: Gene ID: 939083.

4. The genetically engineered bacterium using glucose as the substrate for de novo synthesis of vanillin according to claim 3, wherein
the exogenous transketolase gene is derived from *Escherichia coli* K12;
the exogenous 3-deoxy-7-phosphoheptulonate synthase gene is derived from *Escherichia coli* K12;
the O-methyltransferase gene is derived from *Rattus norvegicus*;
the carboxylic acid reductase gene is derived from *Nocardia iowensis*;
the 4'-phosphopantetheinyl transferase gene is derived from *Mycobacterium marinum*;
the 5-methyltetrahydropteroyltriglutamate-homocysteine methyltransferase gene is derived from endogenous *Corynebacterium glutamicum*;
the S-adenosylmethionine synthase gene is derived from the endogenous *Corynebacterium glutamicum*;
the homoserine O-acetyltransferase gene is derived from the endogenous *Corynebacterium glutamicum*;
the adenosylhomocysteinase gene is derived from the endogenous *Corynebacterium glutamicum.*

5. A method for constructing a genetically engineered bacterium using glucose as a substrate for de novo synthesis of vanillin, comprising the following steps:
(1) using *Corynebacterium glutamicum* ATCC13032 as an original strain to knock out *pcaHG*, *van*, *vdh* and *fud* genes, and obtaining modified *Corynebacterium glutamicum*;
(2) expressing the vanillin synthesis module and the methyl cyclic regeneration module in claim 1 in the modified *Corynebacterium glutamicum.*

6. An application of the genetically engineered bacterium of any one of claims 1-4 or the method of claim 5 in producing vanillin.

7. An application of the genetically engineered bacterium of any one of claims 1-4 or the method of claim 5 in increasing a yield of vanillin.

8. A method for producing vanillin, using the genetically engineered bacterium of any one of claims 1-4 or the genetically engineered bacterium constructed by the method of claim 5 for fermentation.
